**Europäisches Patentamt**

**European Patent Office**

**Office européen des brevets**

(11) Publication number : **0 536 059 A2**

## (12) EUROPEAN PATENT APPLICATION

(21) Application number : **92420329.2**

(22) Date of filing : **25.09.92**

(51) Int. Cl.⁵ : **G01N 31/22**

(30) Priority : **30.09.91 US 768787**
**24.08.92 US 934842**

(43) Date of publication of application :
**07.04.93 Bulletin 93/14**

(84) Designated Contracting States :
**AT BE CH DE DK ES FR GB GR IE IT LI LU MC NL PT SE**

(71) Applicant : **EASTMAN KODAK COMPANY**
**343 State Street**
**Rochester, New York 14650-2201 (US)**

(72) Inventor : **Yeaw, David C., c/o EASTMAN KODAK COMPANY**
**Patent Legal Staff, 343 State Street**
**Rochester, New York 14650-2201 (US)**
Inventor : **Hoffmann, Andrew F., c/o EASTMAN KODAK COMPANY**
**Patent Legal Staff, 343 State Street**
**Rochester, New York 14650-2201 (US)**

(74) Representative : **Parent, Yves et al**
**Kodak-Pathé Département Brevets et Licences Centre de Recherches et de Technologie Zone Industrielle**
**F-71102 Chalon-sur-Saône Cédex (FR)**

(54) **Test kit and method for the determination of metal ion concentration in solution.**

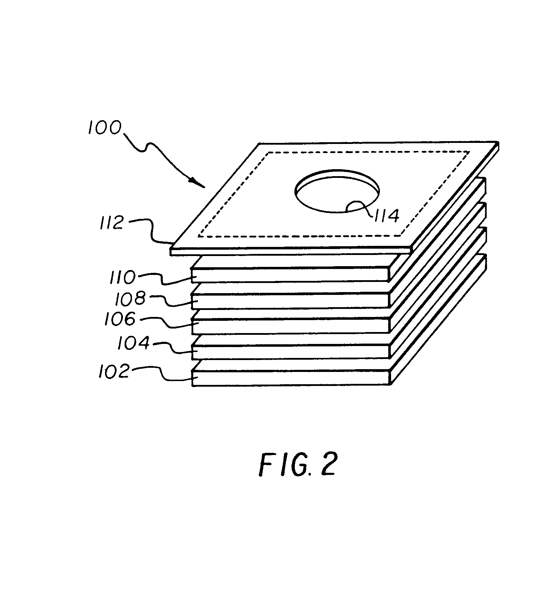

(57) A test kit, composition and method are provided for the colorimetric analysis of the concentration of a metal ion, selected from the group consisting of Group VIB elements, Group VIIB elements, Group IB elements, Group IIB elements, Ni, Pd, and Pt, in an aqueous solution. The composition comprises dithizone, a water-soluble base in an amount sufficient to solubilize the dithizone in an aqueous test solution, and an iron-solubilizing compound. Using a powder composition of the invention, the method of the invention consists of dissolving the base in water to form a first working solution; contacting the first working solution with the remaining reagents to form a second working solution; contacting the test solution with the second working solution; and colorimetrically determining the concentration of the metal ion in the solution. The invention can consist of a test kit containing the above composition, and in addition a multilayered analytical element (FIG. 2) on which the composition reagents are distributed.

FIG. I

## Technical Field

This invention relates to a test kit, composition, and method for colorimetrically determining the concentration of a metal ion in an aqueous solution.

## Prior Art

The discharge of heavy metals, such as silver, is regulated and limited by various codes and statutes, necessitating users of such metals to employ monitoring and/or recovery systems in their operations. It is therefore desirable or necessary for such users to effectively, accurately, and economically monitor metal ion concentrations in effluent streams prior to discharge into the environment.

There are many analytical techniques for determining the presence and/or the concentration of a metal ion, such as silver. One method of obtaining accurate metal ion concentration is by atomic absorption. This requires costly equipment, experienced laboratory technicians, and extensive preparation and test time. Potentiometric titration is another method but also having such disadvantages.

Another techniques for determining the presence or concentration of a metal ion is described in U.S. Pat. 4,920,057. It discloses a method for colorimetric analysis of metals that utilizes dithizone to complex a metal ion from an aqueous solution. It involves, however, forming a biphasic mixture that requires separating and extracting an organic layer from the organic-aqueous biphasic mixture for colorimetric analysis, a procedure that is time-consuming and inefficient.

Other prior art methods and formulations use a metal ion-estimating test paper impregnated with chemicals to estimate the concentration of a ion. U.S. Pat. No. 3,661,532 discloses a test paper impregnated with a mixture of cadmium sulfide and selenium for the colorimetric detection of silver ions. U.S. Pat. No. 3,843,325 discloses a test paper, for semiquantitative, colorimetric detection of metal ions, impregnated with a chelating agent, such as dithizone, and an alkali salt of a weak acid, such as sodium acetate. These, however, are not highly accurate and generally require taking multiple samples to approximate a range of possible concentration.

U.S. Pat. No. 3,972,827 discloses a silver detecting formulation for the colorimetric detection of high purity silver, comprising water, sulfuric acid, and potassium dichromate. A shortcoming is that the formulations are narrowly designed to determine the presence of a single metal ion only at a narrowly defined concentration value.

U.S. Pat. No. 4,309,186 discloses a transparent hydrophilic film having zinc sulfide thereon, that upon ion exchange with a heavy metal ion produces discoloration that is a measurement of the concentration of the heavy metal present. A disadvantage is that it does not provide a quantifiable result.

## Assessment of the Art

It is an object of the invention to provide a quantifiable colorimetric test that yields a single phase solution for determining metal ion concentration in solution.

## Disclosure of Invention

This invention relates to a composition, and a test kit comprising the composition, for the colorimetric measurement of the concentration of a metal ion in solution. the metal ion selected from the group consisting of Group VIB elements, Group VIIB elements, Group IB elements, Group IIB elements, Ni, Pd, and Pt, and the composition comprising the reagents:

(a) dithizone;
(b) a water-soluble base in an amount sufficient to solubilize the dithizone in the solution; and
(c) an iron-solubilizing compound.

The invention also relates to a method of measuring the concentration of the metal ion in the solution, comprising the steps of:

providing the composition described above;
dissolving the base in water to form a first working solution;
contacting the first working solution with the remaining reagents to form a second working solution;
contacting the test solution with the second working solution; and
colorimetrically determining the concentration of the metal ion in the solution.

A useful application of the invention is in a metal recovery process control system for controlling or limiting the discharge of a metal in a waste stream to within an environmentally acceptable limit The system includes process control means for producing a control signal representative of the metal ion concentration value whereby the discharge of the metal ion can be controlled to within a preselected value.

Advantageous Effects of the Invention

The invention provides an easy to use metal ion analysis that does not require extensive effort or time and yields a value accurate over a wide range of metal ion concentrations for a wide variety of metal ions. The invention is useful wherein, although it employs an organic metal-complexing agent such as dithizone as an extractant, a single-phase mixture rather than a two-phase mixture is formed, allowing for direct colorimetric analysis without the necessity of inconvenient, time-consuming separation steps.

Brief Description of the Figures

FIG. 1 is a schematic representation of a silver recovery process control system useful in controlling discharge of an effluent stream to within a desired concentration level of silver ion in the effluent stream.

FIG. 2 is an exploded view of a multilayer analytical element containing the composition of the invention.

FIG. 3 is a graph comparing silver ion concentration of test samples measured by the invention's dithizone analysis to silver ion concentration of the same samples measured by atomic absorption.

FIG. 4 is a graph of absorbance at 470 nm versus reagent age showing the relative stabilities of reagent compositions under different storage conditions and for different composition make-ups.

FIG. 5 is a graph of absorbance at 550 nm versus reagent age showing the relative stabilities of reagent compositions under different storage conditions and for different composition make-ups..

FIG. 6 is a graph of silver concentration versus reagent age using a calibration curve for the colorimeter used for measuring silver concentration.

FIG. 7 is a calibration curve for a Hach DR-700 colorimeter for silver concentrations of 0 to 5 g/L.

FIG. 8 is a calibration curve for a Hach DR-700 colorimeter for silver concentrations of 0 to 1000 mg/L.

FIG. 9 is a calibration curve for a Hach DR-700 colorimeter for silver concentrations of 0 to 200 mg/L.

Detailed Account of the Invention

By the term "metal ion" as used herein, with respect to metal ions capable of being measured by the composition and process of the invention, is also intended to be included such metal ions that are complexed with other ions. For example, $Ag(S_2O_3)_2^{-3}$, $Ag(S_2O_3)_2^{-}$, and $Ag(S_2O_3)_2^{-5}$ are considered to be within the scope of the invention, as are also other complexes of metal ions selected from the group consisting of Group VIB elements, Group VIIB elements, Group IB elements, Group IIB elements, Ni, Pd, and Pt. The periodic table groups designated herein for metal ions whose concentration is measurable by the invention are as shown in the periodic table on the inside front cover of Perry's Chemical Engineers' Handbook, 6th ed., incorporated herein by reference.

The water-soluble base should be present in an amount sufficient to solubilize the dithizone in the aqueous test solution. Generally, this requires an aqueous composition pH of between about 9 and about 12. A preferred such base is a water-soluble base of a Group IA or IIA element. One skilled in the art can select such a base useful in the invention, which include sodium hydroxide, potassium hydroxide, and trisodium phosphate, to name just a few. The composition can comprise liquid reagents or powder reagents as is further addressed below. A solid, non-deliquescing base such as trisodium phosphate or tetrasodium pyrophosphate is preferred in the powder composition over a base such as sodium hydroxide that has a tendency to deliquesce.

Preferably, the base is not contacted with the dithizone prior to use in a test Dithizone tends to rapidly deteriorate in the presence of the base, which can lead to a significant reduction in the test composition shelf life and a decrease in test effectiveness. Accordingly, the base can be packaged or provided separately from the dithizone; the dithizone can be packaged with any of the other reagents of the invention. To improve the flowability of the base in its powder form, the base can be packaged with an anti-caking agent, such as silica or a silicate or a reagent of the invention other than dithizone, such as the iron-solubilizing compound and the dispersant.

The composition also contains an iron-solubilizing compound for controlling iron precipitation from the solution being tested. Iron precipitation can otherwise interfere with and prevent accurate colorimetric measurement of the test solution. Any chemical compatible with the reagents in the composition and capable of maintaining iron solubility at the alkaline pH solution test conditions can suffice if it does not interfere with the viability of the test. Iron-solubilizing compounds useful in the invention include sodium gluconate; water-soluble salts of organic acids, such as: gluconic; citric; and amino-N-acetic acids such as ethylenediamine-N,N,N',N'-tetraacetic, nitrilotriacetic, and the like; and amines such as triethylene tetraamine and triethanolamine, to name but a few.

A preservative agent can optionally be included in the composition of the invention to prolong the shelf life of the composition. In the presence of an alkali solid, dithizone can deteriorate rapidly absent the preservative agent. Readily oxidized compounds or oxygen scavengers are useful as preservatives as long as they do not interfere with the test results. Useful preservative agents include sodium sulfite, sodium bisulfite, ascorbic acid, and sodium metabisulfite; sodium bisulfite is particularly useful in prolonging composition shelf life.

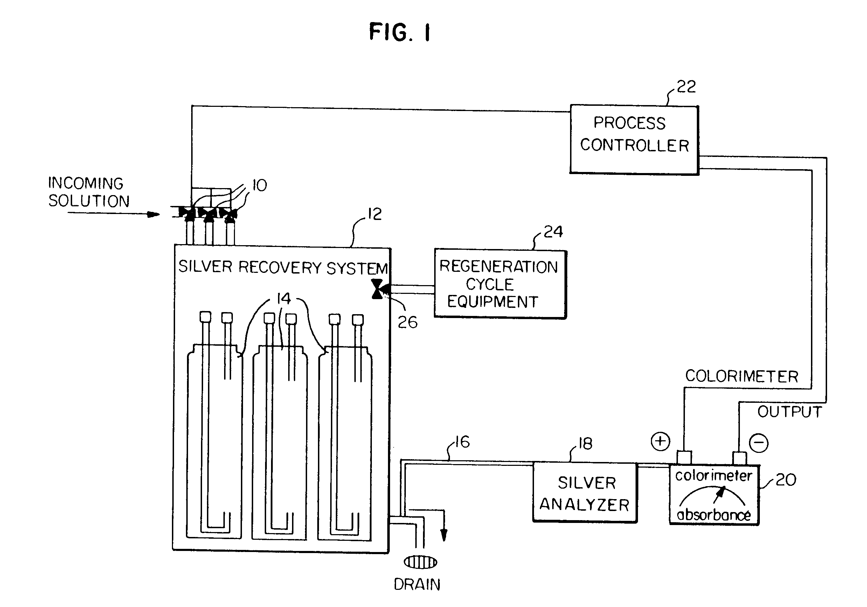

The composition of the invention can also optionally include a dispersant, which is preferred for compositions used in an on-line metal analysis system such as that shown in FIG. 1 and further described below. A dispersant can improve test solution stability and improve the test sensitivity. Useful dispersants include anionic surfactants, such as Versa-TL 73, manufactured by National Starch and Chemical Co., Bridgewater, NJ, and polyoxyethylene alkyl aryl ethers, as well as other nonionic surfactants.

In one embodiment, the composition of the invention comprises an aqueous solution comprising from about 0.001 weight percent to about 0.005 weight percent dithizone, from about 0.2 weight percent to about 2 weight percent base, from about 0.2 weight percent to about 2 weight percent preservative agent, from about 0.005 weight percent to about 0.2 weight percent dispersing agent, and from about 0.2 weight percent to about 2 weight percent iron-solubilizing agent.

In another embodiment, the composition comprises an aqueous solution comprising about 0.002 weight percent dithizone, about 1 weight percent base, about 1 weight percent preservative agent, about 0.05 weight percent dispersing agent, and about 1 weight percent iron-solubilizing agent. A preferred such composition comprises about 0.0015 weight percent dithizone, about 1.2 weight percent sodium hydroxide, about 1 weight percent sodium sulfite, about 0.04 weight percent dispersing agent, and about 1 weight percent sodium gluconate.

The powder composition in one embodiment comprises from about 0.005 to about 0.2 weight percent dithizone, from about 30 to about 90 weight percent base, from about 2 to about 30 weight percent preservative agent, and from about 2 to about 30 weight percent iron-solubilizing compound. In another embodiment, the powder composition comprises from about 0.01 to about 0.05 weight percent dithizone, from about 70 to about 80 weight percent base, from about 10 to about 15 weight percent preservative agent, and from about 10 to about 15 weight percent iron-solubilizing compound.

A test kit of this invention includes the composition described above as well as one or more other reagents, test devices or utensils for performing the test. As stated above, the base is preferably packaged separately from the dithizone to maintain good test composition stability and shelf life.

The test kit can include a test device for colorimetrically determining silver concentration, examples of which are discussed below and in the examples. For carrying out the test, the test kit can also comprise a water-insoluble substrate or multilayer analytical element, such as is described in U.S. 3,992,158, 4,050,898, 4,066,403, 4,144,306, and 4,258,001. In one embodiment of the invention illustrated in FIG. 2, the reagents are distributed within layers on a multilayer analytical element, such as a dry reagent test slide. The dry reagent slide should have at least two reagent layers and can optionally have a top spreading layer. As shown, slide 100 comprises support 102, on which is coated reagent layers 104, 106, and 108. Support 102 can comprise a photographic film support material such as poly (ethylene terephthalate). Each reagent layer can comprise a permeable, hydrophilic material such as gelatin, gelatin derivatives, cellulose derivatives, a polysaccharide such as dextran, gum arabic, agarose and the like, and also synthetic substances such as water-soluble polyvinyl compounds like poly (vinyl alcohol) and poly (vinyl pyrrolidone), acrylamide polymers, etc. Organophilic materials such as cellulose esters and the like can also be useful, and the choice of materials can be determined depending on the intended environment and use. Optical considerations can also affect the choice of reagent layer material, as optimal colorimeter measuring accuracy can depend on minimal optical degradation and effects, such as opacity, contributed by the material.

Optional spreading layer 110 can be useful to uniformly distribute or meter a test sample to the underlying reagent layers. The spreading layer can comprise an isotropic porous layer, selected to be chemically inert to sample components under analysis, such as is described in the above patents on multilayered analytical elements. Useful such spreading layer materials include diatomaceous earth, isotropically porous polymers, and microcrystalline colloidal materials such as microcrystalline cellulose. Support 102 is affixed to mount 112 with reagent layers 104, 106, and 108 positioned therebetween as shown. Mount 112 has aperture 114 therein for receiving the solution to be tested. The dithizone should be in layer 104 because the reactions in the top-most layer or layers should first take place in order to adjust pH and remove substances otherwise interfering with the test. Layer 108 contains the water-soluble base, which is separate from the other reagents of the composition as is discussed above. Layer 106 contains the iron-solubilizing compound, although alternatively it can be distributed in layer 104 with the dithizone; that is, the iron-solubilizing compound can be contained in a

4

third reagent layer or in the same layer as the dithizone. In practicing the invention providing a dry reagent test slide as described, a sample of a test solution is applied via aperture 114 to contact spreading layer 110. Sufficient time is allowed for the sample to diffuse to layer 104 and react with the dithizone, after which the slide is colorimetrically analyzed to determine the concentration of the metal ion in the test solution. Reflection densitomoters useful in analyzing the slide can comprise sophisticated laboratory devices, as described below and in the Examples, as well as devices like the Kodak Ektachem™ DT-60, or versions of the DT-60, programmed with the appropriate software for accepting and reading the slide of the invention.

The present invention provides a method of measuring the concentration of a metal ion, selected from the group consisting of Group VIB elements, Group VIIB elements, Group IB elements, Group IIB elements, Ni, Pd, and Pt, in an aqueous solution using the above test composition. The method of the invention is particularly useful in determining the concentration of a metal ion selected from the group consisting of Cr, Ni, Cu, Ag, Zn, Cd, and Hg, and for determining silver ion concentration in a waste photoprocessing solution. Such determination may be necessary prior to discharging to the environment used developers, fixes, and bleach-fix solutions and the like, and is also useful in silver recovery processes.

Using the powder composition of the invention, the method is carried out by dissolving the base in water to form a first working solution. The first wording solution is then contacted with the remaining reagents to form a second wording solution. A sample of the test solution is contacted with the second working solution to form a single phase product solution, and the concentration of the metal ion in the test solution is determined by colorimetric analysis of the product solution. One of ordinary skill can select the appropriate test equipment capable of accepting the expected volumes of reagents and solution sample, which in the use of the colorimeter equipment can accord with the manufacturer's recommendation or employ the apparatus supplied with the colorimeter by the manufacturer.

After forming the described test solution, it is compared in color intensity to a color standard, such as that for a commercially available colorimeter, the sensitivity of which depends on the liquid path-length along which solution color is viewed. For example, a 15 mm path-length provides greater sensitivity than a 10 mm path-length. Any such colorimeter is suitable in the practice of the invention; although colorimeters can vary in sensitivity and performance, each manufacturer's colorimeter is standardized to provide uniform measurements. Examples of colorimeters useful in the practice of the invention include Perkin-Elmer Model 320 (1-100 mm cell path-length), Bausch & Lomb Spectronic 21 (1-15 mm cell path-length), Hach DR 2000 (12-30 mm cell path-length), Chemtrix 20 (15 mm cell path-length),Chemtrix 20A (15 mm cell path-length), and Technicon (8-50 mm cell path-length). Generally, the longer the cell path-length, the more sensitive the instrument, which allows the testing of a smaller test sample volume or a lower metal ion level.

Using the aqueous composition of the invention, the method comprises the steps of:

providing a contacting zone for receiving the test solution;

introducing into the contacting zone a predetermined sample volume of the test solution and the aqueous composition comprising the above-described reagents;

substantially mixing the test solution and the aqueous composition to form a single-phase product solution; and

measuring the color intensity of the product solution against a known standard to determine the concentration of the metal ion.

The contacting zone that the solutions are mixed in should be substantially transparent to enable the subsequent colorimetry step to be performed on the product solution in the contacting zone, although an opaque contacting zone can be employed and the product solution transferred to a substantially transparent vessel to undergo the colorimetry test The contacting zone can comprise any suitable vessel or cell or the like. For a particular colorimeter, the test solution and aqueous composition solution volumes and/or flow rates can be selected to conform to the volume of the colorimeter's cell. For example, metering means can be employed to control test solution flow and composition solution flow through a continuous on-line colorimeter as is further described below. After forming the single-phase, third solution, it is analyzed colorimetrically as described above for the powder composition.

The amount of aqueous composition needed depends on the amount of test solution and on the estimated concentration of the metal ion to be tested, and is dependent on the selection of the proportion of the reagents of the composition of the invention and on the dilution factor of the first solution. For the above-described preferred aqueous composition of the invention, a preferred volumetric ratio of aqueous composition to first solution is in the range of from about 1000:1 to about 1:1, and a ratio of about 100:1 is particularly preferred.

The invention also comprises a metal recovery process control system for controlling the discharge of a metal ion in a waste stream to within a preselected value, comprising:

means for drawing a sample from a test solution containing a metal ion selected from the group consisting of Group VIB elements, Group VIIB elements, Group IB elements, Group IIB elements, Ni, Pd, and Pt, and controllably introducing the test solution into a contacting zone;

means for introducing the aqueous composition of the invention to the sample of test solution to form a product solution;

means for colorimetrically analyzing the product solution to calculate the value of the concentration of the metal ion in the test solution; and

process control means for producing a control signal representative of such metal ion concentration value whereby the discharge of the metal ion can be controlled to within a preselected value.

The control signal can be employed, for example, to automatically control the discharge of the first solution from a silver recovery system.

FIG. 1 is illustrative of a silver recovery process control system of the invention. A feed solution containing silver ion is introduced through flow control valves 10 to silver recovery system 12. Silver recovery system 12 can comprise any conventional recovery system, for example a plurality of ion exchange columns 14 as depicted therein, or a system employing electrolytic desilvering electrodes, to name but a few. Means for drawing a sample of the first solution, which is the solution undergoing treatment, comprises sampling line 16 connected to a pump, for example a metering pump. Metering or "peristaltic" pumps are well-known in the art and are manufactured , for example, by Technicon Corp. and Manistat Corp. Sampling line 16 feeds the first solution to a contacting zone, silver analyzer 18, which comprises a continuous flow-through cell, a photomultiplier circuit, and selectable-wavelength light-generating means (not illustrated), which altogether function as a standard, continuous on-line colorimeter. Such colorimeter, as is well-known in the art, is means for colorimetrically analyzing the third solution, providing a continuous monitor and control signal output for test solution therethrough. As such, silver analyzer 18 also comprises means for introducing the second solution comprising the test composition of the invention. Such means for introducing the second solution can comprise, for example, standard glass fittings and tubing (not illustrated) for introducing and mixing the first and second solutions to produce the third, test solution. The third solution flows through the colorimeter cell, upon which silver analyzer 18 provides an output value of silver concentration displayed on scale 20. The output value of silver concentration is converted to a representative electrical signal that is provided to process controller 22, where it is compared to a predetermined operator-set value. Process controller 22 then gene.rates at least one control signal for controlling the setting of feed control valves 10 and/or for controlling regeneration cycle equipment 24 which, by means of flow control valve 26, controls the feed-regeneration-discharge cycle of silver recovery system 12. The discharge of treated solution from the silver recovery loop and the concentration of silver in such effluent is thereby monitored and controlled to within acceptable limits.

In practice, the operator can colorimetrically determine the concentration of a single such species of metal ion, or can obtain colorimetry data that results from more than one such species present The absorbance curves are additive, so that a colorimetry measurement employing the method of the invention on a feed solution having more than one such species present will not determine the concentration of a single specie.

The method is applicable, however, when measuring the concentration of a single specie that is present in solution with other such metal ions of known concentration. A baseline reference can be established for the specie or species of known concentration and the contribution of the measured unknown specie thus determined and compensated for. For example, in a solution containing equal amounts of zinc and cadmium, in measuring cadmium concentration, 45 percent of the absorbance measured at the cadmium wavelength (510 nm $I_{max}$) would be contributed by zinc (492 nm $I_{max}$). In a solution containing equal amounts of copper and mercury, in measuring mercury concentration, 25 percent of the absorbance measured at the mercury wavelength (518 nm $I_{max}$) would be contributed by copper (468 nm $I_{max}$). In a solution containing equal amounts of copper and silver thiosulfate complex, in measuring silver thiosulfate complex concentration, 12 percent of the absorbance measured at the silver thiosulfate complex wavelength (545 nm $I_{max}$) would be contributed by copper (468 nm $I_{max}$). Representative absorbance values for these and other metal ions are given in the Examples, below.

The invention is further illustrated by the following examples of its practice. Tests were carried in Examples 1-4 out using liquid reagent mixtures of the composition of the invention to assess the viability and accuracy of the composition and method of the invention. In Examples 5-6, tests were run using the powder compositions of the invention to confirm their stability and efficacy in obtaining accurate metal ion concentration readings.

Example 1

Preparation of Reagent Concentrate:

100 grams of sodium hydroxide were added to about 500 ml of water in a flask, and the solution was stirred and allowed to cool. To this solution was added 15 grams sodium sulfite, 0.75 grams of dithizone diphenylthiocarbazone, formula $C_6H_5N=NCSNHNHC_6H_5$), and 20 ml of Versa-TL 73. The volume was adjusted to 1000 ml and the flask stoppered.

Working Reagent:

To about 400 ml of water was added 5 grams sodium sulfite, 5 grams sodium gluconate, and 5 grams of sodium hydroxide. To the solution was then added 10 ml of Reagent Concentrate. The volume was then adjusted to 500 ml. The wording reagent should be used within 4 hows or a new one prepared thereafter.

Test Procedure:

To a test tube or other appropriate container was introduced 5 ml of wording reagent. To the container was added an amount of sample solution (for example, as suggested in Table 4, below), and the container was stoppered and shaken. A cuvette was then filled with the solution and the absorbance measured at 560 nm (the specific wavelength at which the colorimeter was calibrated).

A Calibration Graph, or alternatively a linear regression chart, was obtained for the particular colorimeter employed, in order to provide means for determining the unknown concentration of metal ion, as follows:

A. Preparation of a 10.00 g/L silver stock solution:

15.74 g silver nitrate was dissolved in about 200 mL water in a one liter volumetric flask. 15 g sodium bromide was dissolved in about 200 mL water in a beaker. The contents of the beaks are poured into the flask while stirring to form a yellow precipitate. 200 mL of 58% ammonium thiosulfate solution was added to the flask and the volume adjusted to 1000 mL after the precipitate dissolves.

B. Preparation of Standard Silver Solutions

In separate 1000 mL volumetric flasks was added an amount of the silver stock solution prepared in step A to thereby prepare standard silver solutions having silver concentrations of: (1) 0.5 mg/L, 1.0 mg/L, and 2.0 mg/L, respectively; (2) 5 mg/L, 10 mg/L, 20 mg/L, and 40 mg/L, respectively; and (3) 50 mg/L, 100 mg/L, 200 mg/L, and 400 mg/L, respectively.

To calibrate the colorimeter, for each such grouping 1-3 above, each solution absorbance was measured, and a blank value also obtained by measuring absorbance of an aliquot of distilled water, which blank value absorbance was subtracted from each sample absorbance obtained. This procedure was carried out on a specific colorimeter to obtain the following typical values, which are broken down into Tables 1-3 for groupings 1-3:

(NOTE- "change in absorbance" as used hereinbelow for each Table 1-3 = Absorbance value for the stated standard concentration value - Absorbance value for concentration at 0 mg/L)

TABLE 1

| [Silver] (mg/L) | Absorbance | Change in Absorbance |
|---|---|---|
| 0 | 0.116 | N/A |
| 0.54 | 0.160 | 0.044 |
| 1.1 | 0.189 | 0.073 |
| 2.1 | 0.230 | 0.114 |

A standard linear regression calculation then provides the unknown silver ion concentration according to the following equation based on the values shown in Table 1:

$$\text{Silver Ion Concentration} = (\text{change in absorbance})(22.4) - 0.5$$

TABLE 2

| [Silver] (mg/L) | Absorbance | Change in Absorbance |
|---|---|---|
| 0 | 0.012 | N/A |
| 5.0 | 0.056 | 0.053 |
| 10 | 0.120 | 0.108 |
| 20 | 0.226 | 0.214 |
| 30 | 0.330 | 0.318 |
| 40 | 0.430 | 0.418 |

A standard linear regression calculation then provides the unknown silver ion concentration according to the following equation based on the values shown in Table 2:

Silver Ion Concentration = (change in absorbance)(96) - 0.3

TABLE 3

| [Silver] (mg/L) | Absorbance | Change in Absorbance |
|---|---|---|
| 0 | 0.011 | N/A |
| 50 | 0.123 | 0.112 |
| 100 | 0.197 | 0.186 |
| 200 | 0.318 | 0.307 |
| 300 | 0.430 | 0.419 |
| 400 | 0.540 | 0.529 |

A standard linear regression calculation then provides the unknown silver ion concentration according to the following equation based on the values shown in Table 3:

Silver Ion Concentration = (change in absorbance)(847) - 54

Similarly, metal ion concentration can be calculated from the calibration curve generated for any particular instrument being used and for the sample size employed. As shown, separate calibration curves may be obtained for different ranges of concentrations. The tester may then vary sample size depending on expected concentration of silver ion, for example, as in Table 4 below.

## TABLE 4

| Expected Silver Concentration | Test Solution Sample Size |
|---|---|
| Less than 5 mg/L | 5 ml |
| 5-100 mg/L | 500 uL |
| 0.1-2.0 g/L | 100 uL |
| Over 2 g/L | 50 uL |

Note- sample sizes are for a cell path length of approximately 1 cm.

Example 2

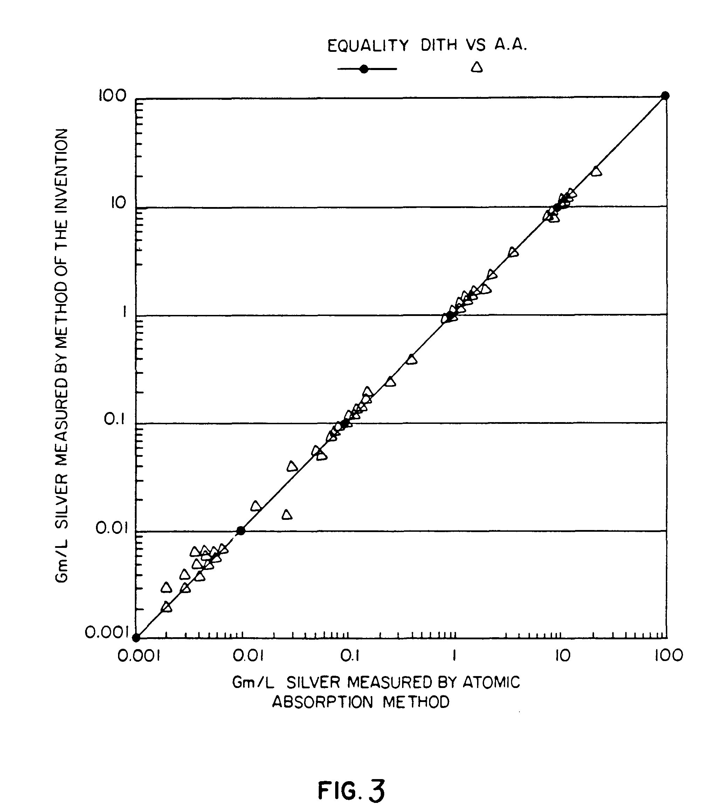

The procedure of Example 1 was carried out on an aqueous solution containing silver ion, and each test solution sample was also tested for silver concentration by the atomic absorption method. The results are shown in Figure 3, from which it can be seen that the method of the invention provides good results that correlate closely to atomic absorption data.

Example 3

The procedure of Example 1 was carried out on an aqueous solution containing silver ion, and each test solution sample was also tested for silver concentration by potentiometric titration with sulfide ion. The results are shown in Table 5, below, from which it can be seen that the method of the invention provides good results that correlate closely to yet another accurate, accepted scientific procedure for measuring concentration.

TABLE 5

| Silver Concentration by Potentiometric Titration (mg/L) | Silver Concentration by Method of the Invention (mg/L) |
|---|---|
| 186 | 181 |
| 236 | 225 |
| 230 | 236 |
| 278 | 278 |
| 308 | 292 |
| 329 | 332 |

Example 4

Relative sensitivities of various metals with dithizone complexation were determined for the purpose of establishing a baseline measurement to compensate for the presence of known concentrations of metal ion species in the same test solution when measuring an unknown metal ion specie. The results, normalized to a 500 mg/L concentration of each metal ion and based on a 5 uL test solution sample in 5 mL of the Working Reagent of Example 1, are shown in Table 6, below.

TABLE 6

| Metal Ion | Wavelength (nm) | Absorbance |
|---|---|---|
| $Cr^{+6}$ | 370 | 0.460 |
| $Cu^{+2}$ | 468 | 0.193 |
| $Zn^{+2}$ | 492 | 0.410 |
| $Ag^{+1}$ | 505 | 0.464 |
| $Cd^{+2}$ | 510 | 0.459 |
| $Hg^{+2}$ | 518 | 0.360 |
| $Ag(S_2O_3)_2^{-3}$ | 545 | 0.538 |
| $Ni^{+2}$ | 660 | 0.155 |

Ions tested showing no color change were Fe, Ca, Pb, Sn, Rb, Al, Zr, and Ba.

The following Examples 5-6 were tests run using a powder composition of the invention.

Example 5

**Mixture 1**

Trisodium phosphate - 0.75 g
Sodium Gluconate - 0. 15 g
Sodium sulfite - 0. 10 g
Dithizone - 0.0003 g

9

Mixture 1 was a one-part mixture and was tested under the following three sets of conditions-Keeping conditions:

**A.** Room temperature, open container.

**B.** Room temperature, closed container.

**C.** Low Temperature (-5° C.)

**Mixture 2**

Mixture 2 was a two-part mixture ("A" and "B"), with the base separated from the other test composition ingredients-Part A.

Water-soluble base: Trisodium phosphate - 0.75 g

Part B.

Sodium Gluconate - 0.15 g
Sodium sulfite - 0.10 g
Dithizone - 0. 0003 g
Keeping conditions:
Room temperature, open container.

**Mixture 3**

Part A.

Trisodium phosphate - 0.75 g (separate)

Part B.

Sodium Gluconate - 0. 15 g
Sodium bisulfite - 0. 10 g
Dithizone - 0.0003 g
Keeping conditions:
Room temperature, closed container.

Mixture 3 was therefore the same two-part mixture as mixture 2, but with different keeping conditions.

Tests were run as follows to test the stability of the powder compositions of the invention. The test was designed to monitor any deterioration of the dithizone in the solid mixture and any decrease in the capacity of the reagent, once prepared for use, to measure silver.

The working reagent was made by adding, in the case of the single mix #1 above, 1 g of solid to 10 mL of deionized water and _gently_ mixing by tipping and swirling to dissolve. In the case of mixtures #2 and #3, the separated solids, each 10 mL of deionized water would receive 0.75 g of the Part A portion and 0.25 g of the Part B portion, followed by the same gentle dissolution.

(Note: If the liquid Part A is used for mixes #2 or #3, 10 mL replaces the deionized water and the Part B is dissolved into that.)

The instruments used for the stability testing compared the HACH DR/700 to the Perkin-Elmer 300 Spectrophotometer.

Several measurements were then taken on the solutions to monitor the stability:

Measurements:

**A.** The absorbance of each reagent solution in a 1-cm cell at 470 nm (the lambda max of the dissolved dithizone) on the Perkin-Elmer spectrophotometer.

**B.** The absorbance of each reagent solution in a 1-cm cell at 550 nm on the Perkin-Elmer spectrophotometer (closest available filter on the HACH DR/700 colorimeter to the lambda max of the silver-dithizone complex).

**C.** The absorbance of each reagent solution after adding 10μL of a 5.0 g/L silver standard in a 1-cm cell at 550 nm on the Perkin-Elmer spectrophotometer.

**D.** The absorbance of the same solutions as measurement "C" above using the HACH DR/700 colorimeter, previously zeroed with the corresponding reagent solution.

From these data, The following calculations were made:

**E.** The absorbance obtained in measurement B above was subtracted from that obtained in measurement C to give the contribution of the silver dithizone complex alone at 550 nm.

**F.** To the absorbance obtained in measurement D above, a previously determined calibration curve (using fresh reagent) was applied. The calibration procedures and data are shown and discussed below.

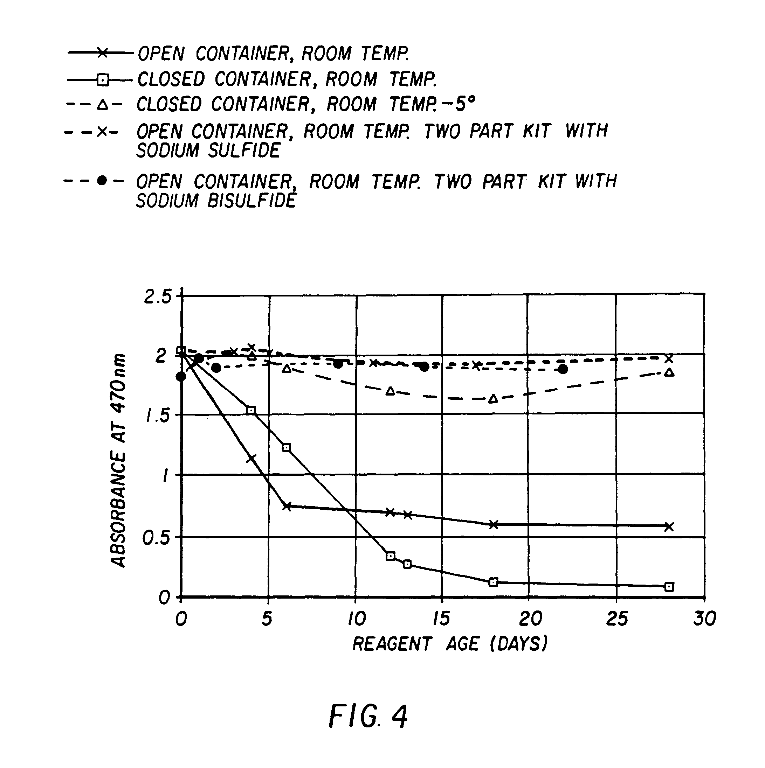

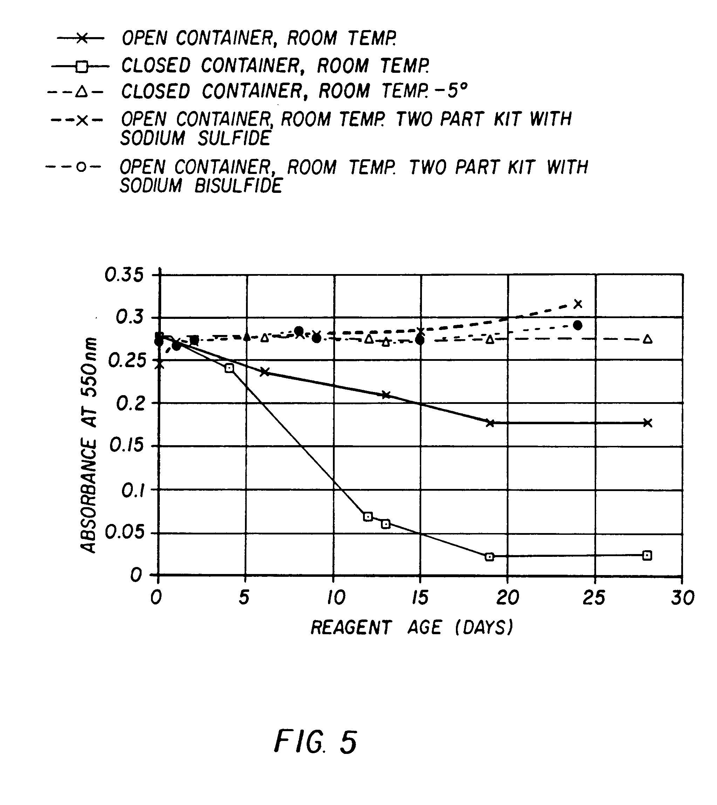

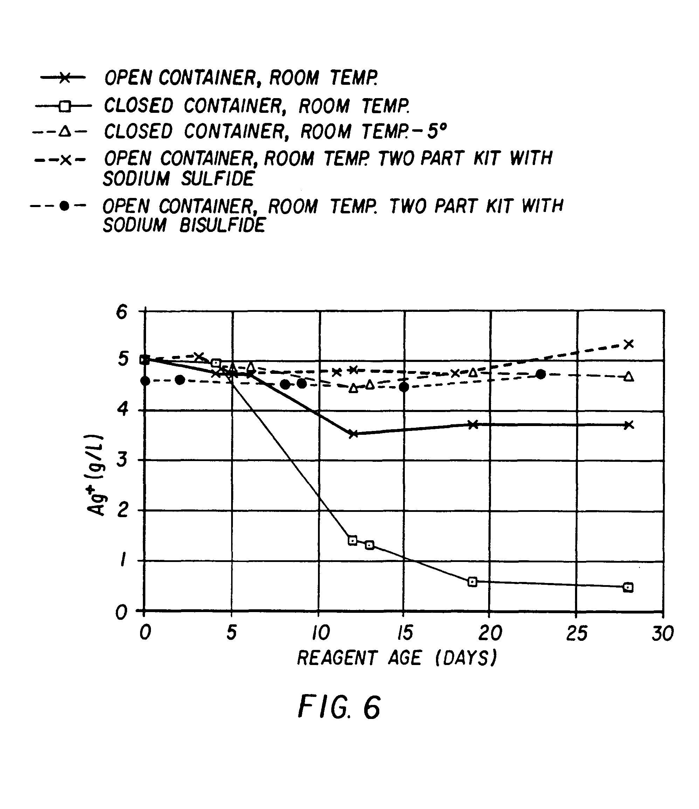

From these data, three sets of data were graphed following the five reagent variations outlined above: Mixture 1- keeping conditions A, B, and C; Mixture 2; and Mixture 3- for dithizone stability (Measurement A), silver complexing capacity (Measurement E), and actual performance on the recommended equipment (Measurement F). These are shown in Figures 4, 5, and 6, respectively.

**The Two-Part Reagent System -**

Option 1: All bulk reagents

The trisodium phosphate can be either dry, to be mixed at about 75 g/L, or as a solution of approximately 75 g/L. The rest of the reagent chemicals can be a blend in the ratio of 15 gluconate: 10 bisulfite: 0.03 dithizone. In some cases 10 parts solid dispersant may be desirable.

Option 2: Bulk phosphate and individual reagent packs.

The Trisodium phosphate can be as in option 1. The reagent packets can each contain 0.10 g of sodium bisulfite, 0.15 g of sodium gluconate, and 0.00003 g of dithizone. In some cases 0.10 g solid dispersant may be desirable.

Option 3: Same as option 2 with measured phosphate.

The phosphate can be premeasured for a certain volume of liquid, i.e.. as a 75 g package to be mixed with 1000 mL of water for use.

Option 4: All individually premeasured single-test packets.

The phosphate can be 0.75 g in one packet. The other chemicals would be as in option #2 above. Any viable variations in ratios concentrations, etc. are claimed.

The test procedures were as follows:

I. PROCEDURE FOR THE ANALYSIS OF SILVER IN FIXER OR BLEACH-FIX (USING LIQUID REAGENT PART A.)

**The equipment used for the test was:**

1. DR/700 HACH Colorimeter
2. Automatic pipettors (10 μL; 50 μL; 200 μL; 5.00 or 10.00 mL)
3. Calibrated Scoop to deliver approximately 0.25 g

**The reagents used were:**

1. Working Solution (Part A., containing the trisodium phosphate)
2. Solid Reagent (Part B., containing the remainder of chemicals necessary for the reagent)

Using the 5.00 or 10.00 mL pipettor, 10 mL of Working Solution were added to a HACH screw-top cuvette. Using the 0.25 g scoop, a level scoop of Solid Reagent was added to the cuvette. The cuvette was covered and mixed until the powder dissolved by gently tilting and swirling the cuvette. [Note- the cuvette should not be shaken). The outside of the cuvette was carefully wiped clean and placed in the sample chamber of the colorimeter with the diamond on the cuvette facing forward, and the colorimeter turned on. The colorimeter was zeroed and the cuvette was removed and uncovered. The proper pipettor was selected (see the chart below) and an aliquot of sample added to the cuvette.

| Estimated Silver Concentration | Pipettor Size |
|---|---|
| 0.5 to 5.0 g/L | 10 μL |
| 100 to 1000 mg/L | 50 μL |
| 20 to 100 mg/L | 200 μL |

The cuvette was covered and it was tilted and swirled as in step 2. to mix. The cuvette was returned to the sample chamber. About 1 to 8 minutes after contacting the sample with the test composition, the absorbance displayed on the LCD was recorded. The color complex is stable for much longer times.

II. PROCEDURE FOR THE ANALYSIS OF SILVER IN FIXER OR BLEACH-FIX

(USING SOLID BUFFER REAGENT PART A.)

**Reagents:**

1. Buffer Reagent (Part A.)
2. Solid Reagent (Part B.)

Using an appropriate pipettor, 10 mL of deionized water was added to a HACH screw-top cuvette. Using a 0.75 g brass scoop, a level scoop of Buffer Reagent was added to a cuvette. The cuvette was cover and the powder mixed until dissolved by gently tilting and swirling the cuvette. Using a 0.25 g Brass Scoop, a level scoop of Solid Reagent was added to the cuvette. The cuvette was covered and mixed until the powder dissolved by gently tilting and swirling the cuvette. [Note- the cuvette should not be shaken). The outside of the cuvette was wiped clean and placed into the sample chamber of the colorimeter with the diamond forward. The colorimeter was zeroed and then the cuvette removed and uncovered. An aliquot of sample was added to the cuvette. The cuvette was covered, and tilted and swirled as in step 2. to mix. The cuvette was then placed into the sample chamber and as before after about 1 to 8 minutes the displayed absorbance recorded.

**Calculations:**

The metal ion concentration is calculated from a previously established calibration curve that can be established as below:

**Calibration Procedure for Silver in Fixers and Bleach-Fixes Using the Solid Reagent**

The same procedure was followed for both fixers and bleach-fixes to set up standard solutions, analyze these solutions, and produce linear regression calibration curves from the absorbance data obtained.

The matrix used for the fixer standards was the fresh Kodak RA-3000 Graphic Arts Fixer. That used for the bleach-fix was a fresh Kodak Process RA-4 Bleach-Fix NR.

Two stock solutions containing 10.0 g/L silver ion were mixed in the two matrices using the following procedure:

1. 3.14 g of $AgNO_3$ was dissolved in a minimum (about 10 mL) of deionized water in a 200 mL volumetric flask.
2. 3 g of NaBr was dissolved in a small beaker as above.
3. After dissolution, the bromide solution was added to the volumetric flask to make AgBr precipitate.
4. The volumetric flask was adjusted to volume with the proper matrix solution and mixed to dissolve the AgBr.

Three sets of standard solutions were mixed to cover the silver ion concentration ranges of 50 to 500 mg/L, 100 to 1000 mg/L, and 0.5 to 5.0 g/L. These were all dilutions of the 10.0 g/L stock solutions. Each of the standard solutions was analyzed using the procedures described above. The following is a listing of the standard series with the absorbances obtained:

## TABLE 7

| Concentration | Pipettor | Matrix | Absorbance |
|---|---|---|---|
| 5.0 g/L | 10 μL | Fixer | 0.481 |
| 2.0 g/L | 10 μL | Fixer | 0.194 |
| 1.0 g/L | 10 μL | Fixer | 0.096 |
| 0.5 g/L | 10 μL | Fixer | 0.046 |
| | | | |
| 1000 mg/L | 50 μL | Fixer | 0.487 |
| 500 mg/L | 50 μL | Fixer | 0.246 |
| 200 mg/L | 50 μL | Fixer | 0.101 |
| 100 mg/L | 50 μL | Fixer | 0.054 |
| | | | |
| 500 mg/L | 200 μL | Fixer | (not read) |
| 200 mg/L | 200 μL | Fixer | 0.390 |
| 100 mg/L | 200 μL | Fixer | 0.191 |
| 50 mg/L | 200 μL | Fixer | 0.098 |
| | | | |
| 5.0 g/L | 10 μL | Bleach-Fixer | 0.462 |
| 2.0 g/L | 10 μL | Bleach-Fixer | 0.176 |
| 1.0 g/L | 10 μL | Bleach-Fixer | 0.081 |
| 0.5 g/L | 10 μL | Bleach-Fixer | 0.034 |

## TABLE 7 (continued)

| Concentration | Pipettor | Matrix | Absorbance |
|---|---|---|---|
| 1000 mg/L | 50 μL | Bleach-Fixer | 0.465 |
| 500 mg/L | 50 μL | Bleach-Fixer | 0.229 |
| 200 mg/L | 50 μL | Bleach-Fixer | 0.090 |
| 100 mg/L | 50 μL | Bleach-Fixer | 0.041 |
| | | | |
| 500 mg/L | 200 μL | Bleach-Fixer | (not read) |
| 200 mg/L | 200 μL | Bleach-Fixer | 0.380 |
| 100 mg/L | 200 μL | Bleach-Fixer | 0.184 |
| 50 mg/L | 200 μL | Bleach-Fixer | 0.096 |

The data from the first two sets in each case (.5 to 5 and 100 to 1000) were considered essentially duplicates, since the sample size was increased by 5X and the concentration was decreased 5X. These data were therefore combined as a single data set to obtain the regression data at each level. (To calculate the higher set, the 1.0 was scaled up to 5.0, the .5 was scaled up to 2.5, etc. The reverse was done to calculate the lower set.)

The linear regressions obtained were as follows:

Fixer using the 10 μL pipettor -

(10.4)*(Abs.) = g/L Silver $r^2$ =.9996

Fixer using the 50 μL pipettor-

(2070)*(Abs.) = mg/L Silver $r^2$ =.9996

Fixer using the 200 μL pipettor-

(512)*(Abs.) = mg/L Silver $r^2$ = .9992

Bleach-Fix using the 10 μL pipettor-

(10.6)*(Abs.)+0.1 = g/L Silver $r^2$ = .9996

Bleach-Fix using the 50 μL pipettor-

(2112)*(Abs.)+21 = mg/L Silver $r^2$ =.9996

Bleach-Fix using the 200 μL pipettor-

(525)*(Abs.)+1 = mg/L Silver $r^2$ =.9992

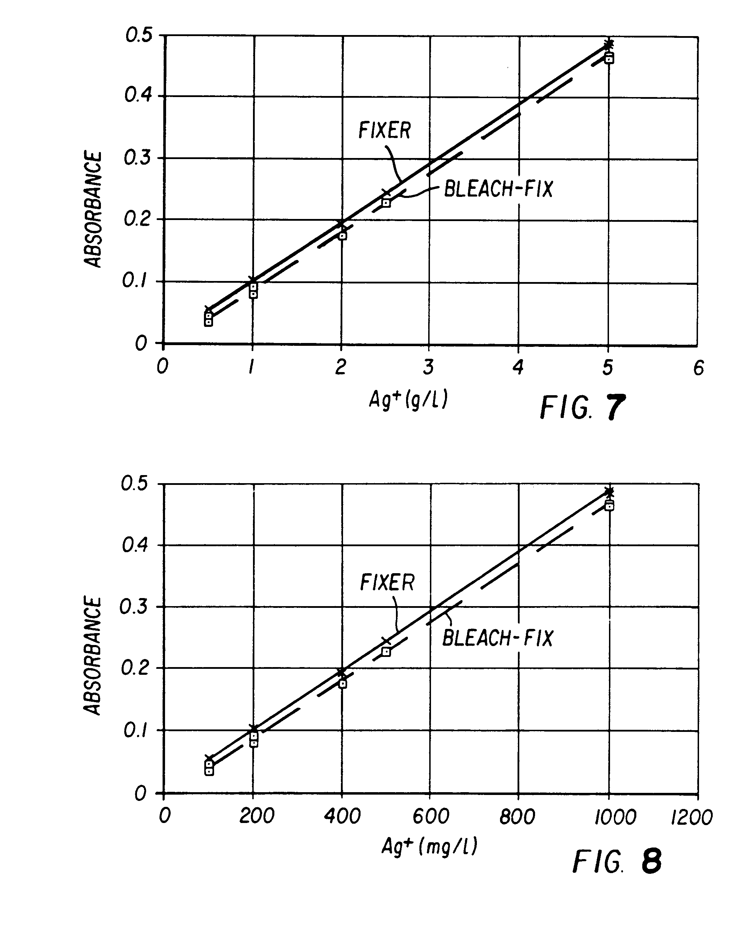

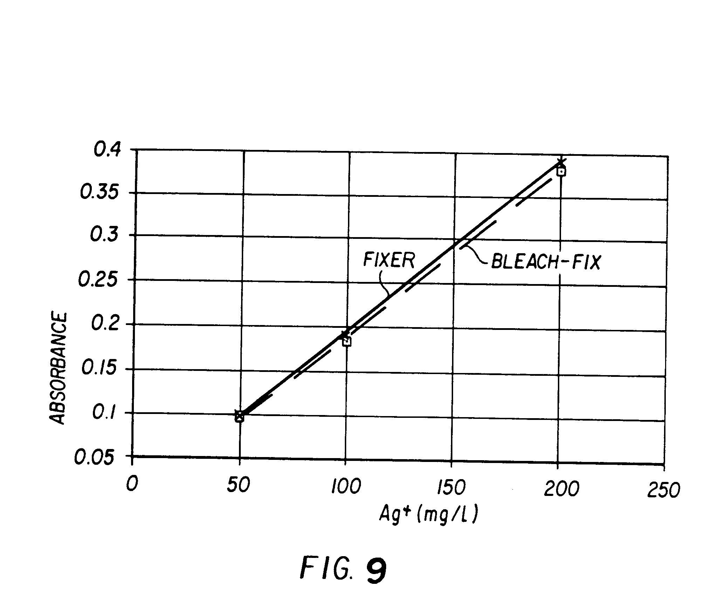

The calibration data are graphically shown in figures 7, 8, and 9.

The results show the precision and accuracy obtained by the simple techniques of the invention, which can be readily used by non-laboratory operators. The results further show the stability of the dry, blended reagents.

Example 6

A silver test reagent was prepared by weighing and blending the following components:

Sodium phosphate, tribasic, dodecahydrate 71.31 g
Sodium gluconate 5.00 g
Dithizone 0.0208 g
Iconol NP-100 surfactant (BASF Corporation) 10.00 g

A series of silver standards and samples of Process RA-4 bleach fixer containing various concentrations of silver were prepared and analyzed with the test kit. Silver standards consisted of silver nitrate at known concentrations in water. Fresh bleach fixer solutions were prepared according to the standard formula and contained known amounts of silver added as silver bromide. A seasoned bleach fixer solution was obtained from a processing machine, and was analyzed as received and with a known addition of silver bromide. The following procedure was used for the analyses:

1. A graduated 2.54-cm colorimeter cell is filled to the 10-mL mark with distilled or deionized water.

2. A pre-weighed portion (0.86 g) of the silver test reagent described above is added to the water in the cell and dissolved. The solution is allowed to stand for 2 minutes to reach a stable absorbance.

3. The cell is placed into a digital colorimeter equipped with a nominal 550 nm filter and the colorimeter is adjusted to read zero absorbance.

4. For samples containing 0.1 to 2.0 g/L silver, 50μL of sample are added to the cell, and the solution is gently mixed.

5. After a 6 minute waiting period for full color development, the cell is placed into the colorimeter and the absorbance is measured.

6. For samples containing 2 to 20 g/L silver, an identical procedure is followed, except that 5μL of sample are used.

7. A calibration curve is obtained by linear regression of the absorbances of the standards.

8. The absorbances of the test solutions prepared from each bleach fixer solution are then converted to silver concentrations in the bleach fixer solutions via the calibration equation.

In this manner, each bleach fix solution was analyzed four times with the test kit. The following data summarize the results and are compared to results obtained by a conventional atomic absorption spectrophotometric (AAS) method, which was performed in triplicate on each sample:

TABLE 8

| Solution | TEST KIT | | AAS | |
|---|---|---|---|---|
| | $Ag^+$, g/L (Avg) | $1\sigma$ | $Ag^+$, g/L (Avg) | $1\sigma$ |
| Bleach fixer, fresh, 0.51 g/L $Ag^+$ | 0.60 | 0.006 | 0.56 | 0.001 |
| Bleach fixer, fresh, 0.99 g/L $Ag^+$ | 0.95 | 0.007 | 1.13 | 0.006 |
| Bleach fixer, fresh, 1.47 g/L $Ag^+$ | 1.53 | 0.003 | 1.63 | 0.006 |
| Bleach fixer, fresh, 1.48 g/L $Ag^+$ | 1.62 | 0.048 | 1.65 | 0.012 |

## TABLE 8 (Continued)

| Solution | Ag$^+$, g/L (Avg) | 1$\sigma$ | Ag$^+$, g/L (Avg) | 1$\sigma$ |
|---|---|---|---|---|
| Bleach fixer, fresh, 4.93 g/L Ag$^+$ | 5.97 | 0.067 | 5.36 | 0.006 |
| Bleach fixer, fresh, 4.98 g/L Ag$^+$ | 6.20 | 0.12 | 5.32 | 0.17 |
| Bleach fixer, fresh, 7.89 g/L Ag$^+$ | 8.31 | 0.18 | 8.56 | 0.19 |
| Bleach fixer, fresh, 11.4 g/L Ag$^+$ | 12.5 | 0.43 | 12.8 | 0.058 |
| Bleach-fixer, Seasoned, as received | 8.60 | 0.13 | 8.9 | 0.30 |
| Bleach fixer seasoned, with 1.72 g/L Ag$^+$ added | 10.0 | 0.23 | 11.1 | 0.00 |

The results show the good accuracy and precision of the test method and composition of the invention, and display good agreement with silver concentration as measured by atomic absorption.

The invention has been described in detail with particular reference to a preferred embodiment thereof, but it will be understood that variations and modifications can be effected within the spirit and scope of the invention.

## Claims

1. A composition for the colorimetric measurement of the concentration of a metal ion in solution, the metal ion being selected from the group consisting of Group VIB elements, Group VIIB elements, Group IB elements, Group IIB elements, Ni, Pd, and Pt, the composition comprising the reagents dithizone and an iron-solubilizing compound; and wherein the composition further comprises a water-soluble base in an amount sufficient to solubilize the dithizone in the solution.

2. The composition of Claim 1, wherein the water-soluble base is a water-soluble base of a Group IA or IIA element.

3. The composition of Claim 2, further comprising a dispersing agent.

4. The composition of Claim 2, further comprising a preservative agent.

5. The composition of Claim 4, wherein the preservative agent is sodium sulfite, sodium bisulfite, or sodium metabisulfite.

6. The composition of Claim 5, wherein the composition is a powder comprising from about 0.005 to about 0.2 weight percent dithizone, from about 30 to about 90 weight percent base, from about 2 to about 30 weight percent preservative agent, and from about 2 to about 30 weight percent iron-solubilizing compound.

7. The composition of Claim 6, wherein the composition comprises from about 0.01 to about 0.05 weight percent dithizone, from about 70 to about 80 weight percent base, from about 10 to about 15 weight percent preservative agent, and from about 10 to about 15 weight percent iron-solubilizing compound.

8. The composition of Claim 5, wherein the composition is aqueous and comprises from about 0.001 weight percent to about 0.005 weight percent dithizone, from about 0.2 weight percent to about 2 weight percent base, from about 0.2 weight percent to about 2 weight percent preservative agent, from about 0.005 weight percent to about 0.2 weight percent dispersing agent, and from about 0.2 weight percent to about 2 weight percent iron-solubilizing compound.

9. A test kit for the colorimetric measurement of the concentration of a metal ion in solution, the metal ion being selected from the group consisting of Group VIB elements, Group VIIB elements, Group IB elements, Group IIB elements, Ni, Pd, and Pt, comprising the reagents dithizone and an iron-solubilizing compound; and wherein the test kit further comprises a water-soluble base in an amount sufficient to solubilize the dithizone in the solution.

10. The test kit of Claim 9, wherein the base is separately packaged from the dithizone.

11. The test kit of Claim 10, further comprising a preservative agent.

12. The test kit of Claim 11, wherein the reagents are present in the relative weight proportions of from about 0.005 to about 0.2 weight percent dithizone, from about 30 to about 90 weight percent base, from about 2 to about 30 weight percent preservative agent, and from about 2 to about 30 weight percent iron-solubilizing compound.

13. The test kit of Claim 12, wherein the reagents are present in the relative weight proportions of from about 0.01 to about 0.05 weight percent dithizone, from about 70 to about 80 weight percent base, from about 10 to about 15 weight percent preservative agent, and from about 10 to about 15 weight percent iron-solubilizing compound.

14. The test kit of Claim 9, further comprising a dry reagent slide (100) having a top spreading layer (110) and at least two reagent layers (104, 106), and wherein the layer positioned furthest from the spreading layer (110) contains the dithizone, and the water-soluble base is contained in a layer not containing another of the reagents (108).

15. A method of measuring the concentration of a metal ion in an aqueous solution, said metal ion being selected from the group consisting of Group VIB elements, Group VIIB elements, Group IB elements, Group IIB elements, Ni, Pd, and Pt, the method comprising the steps of:
    providing a powder composition comprising the reagents dithizone and an iron-solubilizing compound, and wherein the composition further comprises a water-soluble base in an amount sufficient to solubilize the dithizone in the solution;
    dissolving the base in water to form a first working solution;
    contacting the first working solution with the remaining reagents to form a second working solution;
    contacting the test solution with the second working solution; and
    colorimetrically determining the concentration of the metal ion in the solution.

16. The method of Claim 15, wherein the composition further comprises a preservative.

17. The method of Claim 16, wherein the composition comprises from about 0.005 to about 0.2 weight percent dithizone, from about 30 to about 90 weight percent base, from about 2 to about 30 weight percent preservative agent, and from about 2 to about 30 weight percent iron-solubilizing compound.

18. The method of Claim 17, wherein the composition comprises from about 0.01 to about 0.05 weight percent dithizone, from about 70 to about 80 weight percent base, from about 10 to about 15 weight percent preservative agent, and from about 10 to about 15 weight percent iron-solubilizing compound.

19. A metal recovery process control system for controlling the discharge of a metal ion in a waste stream to within a preselected value, comprising:

means for drawing a sample (16) from a test solution containing a metal ion selected from the group consisting of Group VIB elements, Group VIIB elements, Group IB elements, Group IIB elements, Ni, Pd, and Pt, and controllably introducing the test solution into a contacting zone (18);

means for introducing an aqueous solution ( 18) to the sample of test solution to form a product solution, the aqueous solution comprising dithizone, a water-soluble base in an amount sufficient to solubilize the dithizone in the solution, and an iron-solubilizing compound;

means for colorimetrically analyzing the product solution (20) to calculate the value of the concentration of the metal ion in the test solution; and

process control means (22) for producing a control signal representative of such metal ion concentration value whereby the discharge of the metal ion can be controlled to within a preselected value.

# FIG. I

EP 0 536 059 A2

FIG. 2

EQUALITY DITH VS A.A.

**FIG. 3**

FIG. 4

—✕— OPEN CONTAINER, ROOM TEMP.

—□— CLOSED CONTAINER, ROOM TEMP.

--△- CLOSED CONTAINER, ROOM TEMP. -5°

--✕- OPEN CONTAINER, ROOM TEMP. TWO PART KIT WITH
     SODIUM SULFIDE

--○- OPEN CONTAINER, ROOM TEMP. TWO PART KIT WITH
     SODIUM BISULFIDE

FIG. 5

—×— OPEN CONTAINER, ROOM TEMP.

—□— CLOSED CONTAINER, ROOM TEMP.

--△- CLOSED CONTAINER, ROOM TEMP. - 5°

--×- OPEN CONTAINER, ROOM TEMP. TWO PART KIT WITH
        SODIUM SULFIDE

--•- OPEN CONTAINER, ROOM TEMP. TWO PART KIT WITH
        SODIUM BISULFIDE

FIG. 6

FIG. 7

FIG. 8

FIG. 9